# EUROPEAN PATENT APPLICATION

(11) **EP 4 691 436 A1**
(43) Date of publication of application: **11.02.2026**
(21) Application number: 23930784.6
(22) Date of filing: 23.10.2023
(51) Int. Cl.: A61F 13/15

(54) **INDIVIDUALLY PACKAGED ABSORBENT ARTICLE**

(30) Priority: 30.03.2023 JP 2023055942
(71) Applicant: DAIO PAPER CORPORATION, Shikokuchuo-shi Ehime 799-0492 (JP)
(72) Inventor: KURIHARA, Ryoko, Sakura-shi, Tochigi 329-1411 (JP); KURAMOCHI, Mihoko, Sakura-shi, Tochigi 329-1411 (JP); ITO, Rina, Sakura-shi, Tochigi 329-1411 (JP); IWABUCHI, Haruka, Sakura-shi, Tochigi 329-1411 (JP); TAKAHASHI, Hiromi, Sakura-shi, Tochigi 329-1411 (JP)
(74) Representative: Epping - Hermann - Fischer
(86) International application number: PCT/JP2023/038196
(87) International publication number: WO 2024/202154

(57) **Abstract**

An individually packaged absorbent article in which an absorbent article is individually packaged by a packaging sheet made of a difficult-to-fuse material. A sealing portion is formed by folding the packaging sheet toward an inner surface side of the packaging sheet in a first direction, and sealing both edge portions of a second direction orthogonal to the first direction. The sealing portion includes a plurality of pressed portions which are spaced apart from each other in a surface direction and which are formed by pressing the packaging sheet in a thickness direction. A heat sealing agent is applied in a predetermined pattern to at least a region corresponding to the sealing portion on an inner surface of the packaging sheet, and the pressed portion overlaps the heat sealing agent in a region where the heat sealing agent is applied to the packaging sheet.

## Description

### TECHNICAL FIELD

The present invention relates to individually packaged absorbent articles.

### BACKGROUND ART

Many absorbent articles, such as sanitary napkins, panty liners, incontinence pads, and the like, are provided as individually packaged absorbent articles (individual packages) in a state of being individually packaged and sealed with a packaging sheet for reasons of hygiene during storage, convenience during carrying, and the like. As a packaging structure of the individually packaged absorbent article, for example, a form in which the absorbent article is arranged on the inner surface of the packaging sheet, the packaging sheet is folded to the inner surface side together with the absorbent article, and then both edge portions are sealed is widely known.

Although a resin film, a nonwoven fabric, or the like has been a main material of the packaging sheet, a packaging sheet made of a difficult-to-fuse material, such as paper, is also known as described in, for example, Patent Document 1. In the case where a difficult-to-fuse material is used for the packaging sheet, the packaging sheet cannot be bonded by thermal fusion or cannot be bonded sufficiently, and therefore, it is conceivable to physically deform (forming of irregularities or the like) the packaging sheet to seal the packaging sheet.

### RELATED ART DOCUMENTS

### PATENT DOCUMENT

Patent Document 1: Japanese Unexamined Patent Publication No. 2022-24624

### SUMMARY OF THE INVENTION

### PROBLEMS TO BE SOLVED BY THE INVENTION

However, a seal with sufficient sealing strength cannot be obtained only by physical deformation of the packaging sheet, and sufficient sealing properties of the individually packaged absorbent article may not be obtained. In contrast, for example, sealing with an adhesive, a tape, or the like along both edge portions is also conceivable, but the sealing strength of the sealing portion becomes excessively large, and there is a possibility that the seal cannot be peeled off when the individually packaged absorbent article is opened, or the packaging sheet may be broken when the seal is peeled off.

In view of the above, an object of one aspect of the present invention is to provide a configuration including a sealing portion having an appropriate sealing strength for an individually packaged absorbent article formed using a packaging sheet made of a difficult-to-fuse material.

### MEANS FOR SOLVING THE PROBLEMS

According to one aspect of the present invention, there is provided an individually packaged absorbent article in which an absorbent article is individually packaged by a packaging sheet made of a difficult-to-fuse material, wherein a sealing portion is formed by folding the packaging sheet toward an inner surface side of the packaging sheet in a first direction, and sealing both edge portions of a second direction orthogonal to the first direction, the sealing portion includes a plurality of pressed portions which are spaced apart from each other in a surface direction and which are formed by pressing the packaging sheet in a thickness direction, a heat sealing agent is applied in a predetermined pattern to at least a region corresponding to the sealing portion on an inner surface of the packaging sheet, and the pressed portion overlaps the heat sealing agent applied to the packaging sheet.

### EFFECTS OF THE INVENTION

According to one aspect of the present invention, a configuration is obtained in which a sealing portion having an appropriate sealing strength is provided for an individually packaged absorbent article formed using a packaging sheet made of a difficult-to-fuse material.

### BRIEF DESCRIPTION OF THE DRAWINGS

[FIG. 1] FIG. 1 is a plan view of an individually packaged absorbent article according to an embodiment of the present invention.
[FIG. 2] FIG. 2 is a developed plan view of the individually packaged absorbent article of FIG. 1.
[FIG. 3] FIG. 3 is a sectional view taken along line I-I of FIG. 1.
[FIG. 4] FIG. 4 is a sectional view taken along line II-II of FIG. 1.
[FIG. 5] FIG. 5 is an enlarged view of portion III of FIG. 1.
[FIG. 6] FIG. 6 is a view illustrating a modified example of a pattern of pressed portions of a sealing portion.
[FIG. 7] FIG. 7 is a developed plan view of the individually packaged absorbent article, illustrating a modified example of an application of a heat sealing agent.
[FIG. 8] FIG. 8 is an enlarged view of portion IV of FIG. 2.
[FIG. 9] FIG. 9 is a view illustrating an overlapping relationship between the heat sealing agent and the pressed portions in a first region R1 included in portion V of FIG. 5.
[FIG. 10] FIG. 10 is a view illustrating a modified example of an overlapping relationship between the heat sealing agent and the pressed portions.
[FIG. 11] FIG. 11 is a developed plan view of the individually packaged absorbent article, illustrating a modified example of an application of the heat sealing agent.

### DESCRIPTION OF EMBODIMENTS

Hereinafter, embodiments of the present invention will be described with reference to the drawings. In the drawings, the same or corresponding components are denoted by the same reference numerals unless otherwise specified, and the description thereof may be omitted.

### (Basic Structure of Individually Packaged Absorbent Article)

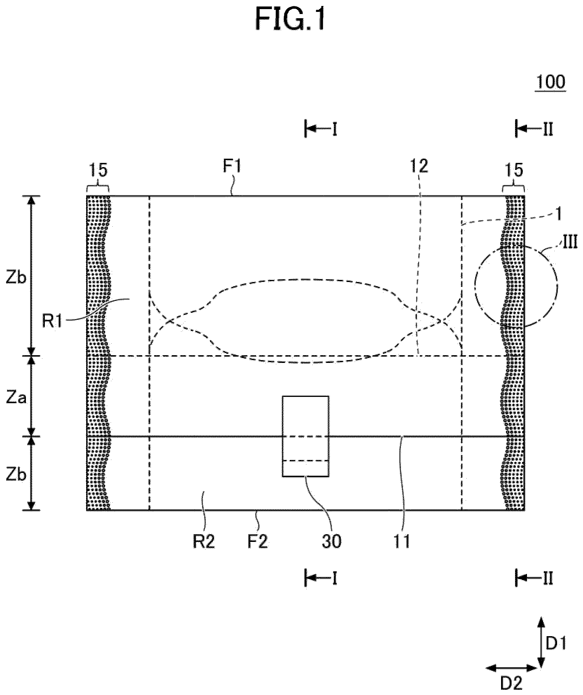

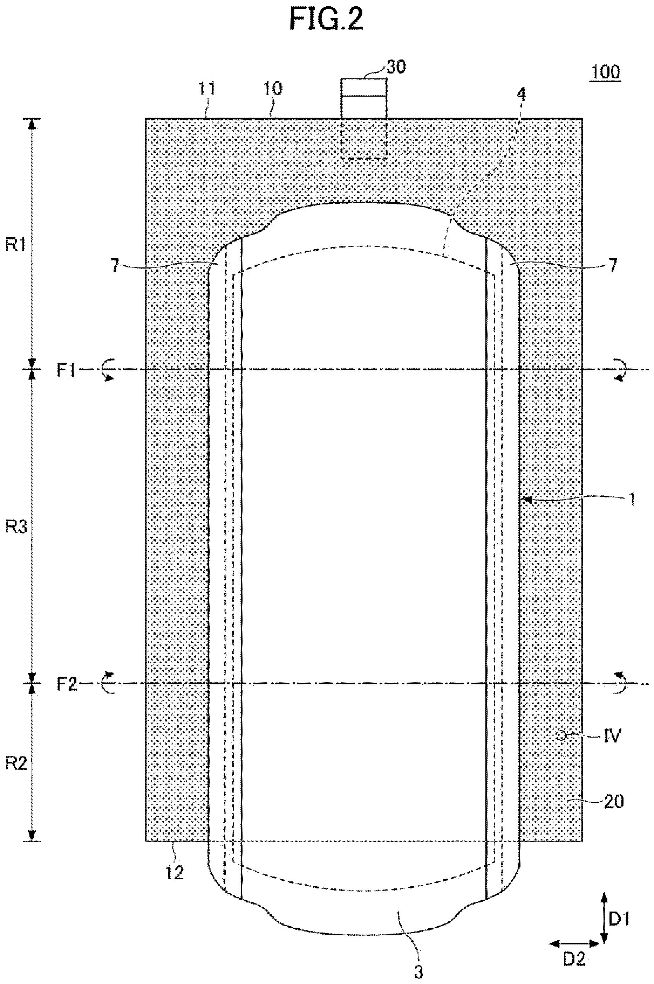

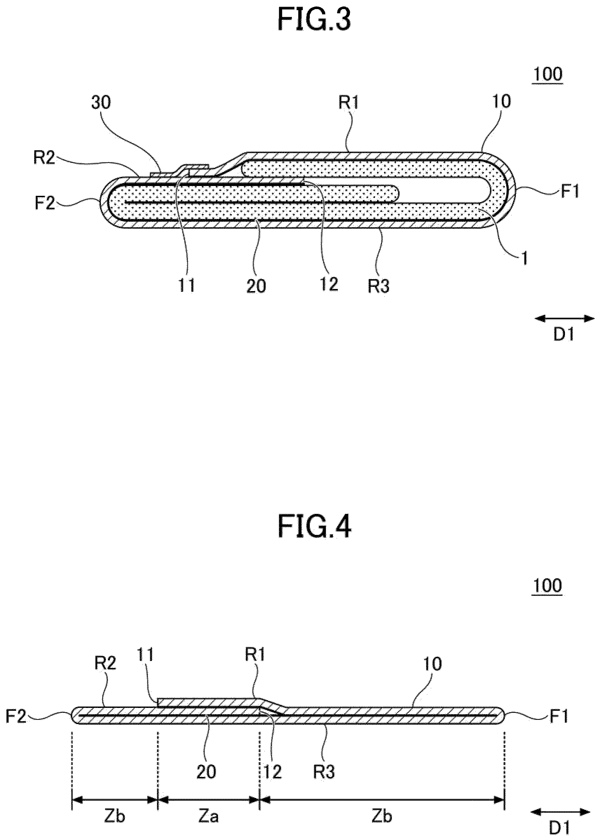

First, a basic structure of the individually packaged absorbent article will be described. FIG. 1 illustrates a plan view of an individually packaged absorbent article 100. FIG. 2 is a plan view of the individually packaged absorbent article of FIG. 1 in a developed state, as viewed from the inner surface of the packaging sheet 10, namely from the skin side of the absorbent article 1. FIG. 3 is a sectional view taken along line I-I of FIG. 1, and FIG. 4 is a sectional view taken along line II-II of FIG. 1.

As illustrated in FIGS. 1 through 4, the individually packaged absorbent article 100 includes a packaging sheet 10 and an absorbent article 1 individually packaged by the packaging sheet 10. As illustrated in FIG. 2, the packaging sheet 10 may have an elongated shape in a developed state, and has a longitudinal direction (first direction) D1 and a lateral direction (second direction) D2 orthogonal to the longitudinal direction. The longitudinal direction D1 and the lateral direction D2 of the packaging sheet 10 may correspond to the longitudinal direction and the lateral direction of the absorbent article 1, respectively.

### (Absorbent Article)

The absorbent article 1 packaged by the packaging sheet 10 may be a flat and elongated article to be worn so as to face a discharge orifice of a body fluid (menstrual blood, vaginal discharge, urine, or the like). Specific examples of the absorbent article 1 may include a sanitary napkin, a panty liner, and a light incontinence pad. In the present specification, the embodiments will be described based on an example in which the absorbent article is a sanitary napkin.

The absorbent article 1 may include, for example, as illustrated in FIG. 2, a liquid permeable top sheet 3, a liquid impermeable back sheet (not illustrated), and an absorber 4 arranged between the top sheet 3 and the back sheet.

As the back sheet, a sheet material having at least water impermeability, such as an olefin-based resin sheet of polyethylene, polypropylene, or the like, can be used. A laminated nonwoven fabric obtained by laminating a nonwoven fabric on a polyethylene sheet or the like, or a laminated sheet of a nonwoven fabric which is substantially liquidimpermeable by interposing a waterproof film therebetween may be used. Alternatively, a material having moisture permeability may be used.

As the top sheet 3, a porous or non-porous nonwoven fabric, a porous plastic sheet, or the like is preferably used. As the material fiber constituting the nonwoven fabric, for example, synthetic fibers, such as olefin such as polyethylene and polypropylene, polyester, and polyamide, regenerated fibers such as rayon and cupra, mixed fibers thereof, and natural fibers such as cotton, can be used alone or in combination of two or more kinds.

The absorber 4 is not limited as long as it is a material capable of absorbing and retaining body fluids, but preferably includes cotton-like pulp and a water-absorbent polymer. As the water-absorbent polymer, superabsorbent polymer granular powder (superabsorbent polymer (SAP)), superabsorbent polymer fiber (superabsorbent fiber (SAF)), and a combination thereof can be used. Examples of the pulp include chemical pulp obtained from wood, cellulose fibers such as dissolving pulp, and artificial cellulose fibers such as rayon and acetate. The pulp may be hardwood pulp obtained from hardwood, softwood pulp obtained from softwood, or a mixed pulp thereof. The pulp may be recycled pulp regenerated from used pulp.

The thickness of the absorber 4 may be 0.5 mm to 25 mm. The absorber 4 may have a structure in which a region facing the body fluid discharge orifice (a body fluid discharge orifice facing region) or a region facing the groove of the buttocks located behind the body fluid discharge orifice facing region is bulged. The absorber 4 has a size and shape that do not allow the absorber 4 to protrude from the top sheet 3 and the back sheet, and at the front and rear end edge portions of the absorber, the outer edges of the back sheet and the top sheet 3 are bonded by an adhesive such as hot melt or by an adhesion means such as heat sealing or ultrasonic sealing.

As illustrated in FIG. 2, in the periphery of the sides of the absorber 4, side sheets 7, 7 may be provided at respective ends of the lateral direction D2, along the longitudinal direction D1. As the side sheet 7, a waterrepellent nonwoven fabric or a hydrophilic nonwoven fabric can be used.

In the example illustrated in FIGS. 1 through 4, the absorbent article 1 is a so-called wingless type, but may be configured as an article having wings extending to the sides. The wings may be formed by bonding the side sheets and the back sheet.

A displacement-prevention adhesive portion for preventing the absorbent article 1 from being displaced from underwear when the absorbent article 1 is attached to the underwear may be provided on the side of the back sheet of the absorbent article 1 (the non-skin side, namely the side facing the underwear when the absorbent article 1 is worn). The displacement-prevention adhesive portion may be formed in a plurality of bands extending in the longitudinal direction D1 or the lateral direction D2.

The total length of the absorbent article 1 can be 140 mm to 430 mm, and the breadth of the absorbent article 1 (the breadth of the main body excluding the wings in the case of having wings) can be 40 mm to 130 mm.

### (Packaging Sheet)

The packaging sheet 10 in the present embodiment may be made of a difficult-to-fuse material. In the present embodiment, the packaging sheet made of a difficult-to-fuse material includes a packaging sheet made of a difficult-to-fuse material and a packaging sheet having a layer of a difficult-to-fuse material on the surface. The difficult-to-fuse material is a material in which packaging sheets cannot be fused or are hardly fused even when heated (for example, heated to 80 °C or higher).

The difficult-to-fuse material may be an aggregate of difficult-to-fuse fibers. The difficult-to-fuse aggregate is preferably paper. The use of the paper packaging sheet 10 can contribute to reduction of plastics and achievement of sustainable development goals. Paper can also provide comfort to users because it can provide a unique texture, such as a gentle look and feel of a natural material. In this specification, "paper" refers to a thin flat sheet obtained by agglutinating plant fibers or other fibers with an agglutinating agent. In particular, "paper" can refer to a sheet containing a plant fiber as a main raw material, for example, one containing 50% or more, preferably 80% or more, of a plant fiber, particularly a cellulose fiber, among the contained fibers. Examples of the plant fiber as a raw material of paper include wood pulp, non-wood pulp, waste paper pulp, and cotton cellulose, and the pulp may be any of mechanical pulp and chemical pulp. The plant fibers may be regenerated fibers such as rayon and cupra, or may be plant fibers recycled from a constituent element of an absorbent article and/or a packaging sheet for an absorbent article (recycled pulp or the like).

Additives may be added to the paper. Specific examples of the paper include various types of paper such as Western paper, Japanese paper, processed paper, and synthetic paper. Further, the paper may be paper conventionally used for other purposes, for example, paper called newsprint paper, printing paper (including high-quality paper), writing paper, drawing paper, packaging paper, tissue paper, hybrid paper, or the like. As thin paper, thin vellum paper, Indian paper, rice paper, glassine paper, tissue paper, toilet paper, filter paper, or the like may be used.

In the present specification, the paper packaging sheet mainly refers to a sheet containing the above-described paper. The paper packaging sheet may include a laminated sheet in which paper and a sheet made of a material other than paper are laminated, as well as a packaging sheet made of only paper. In the case where the packaging sheet 10 includes a sheet made of a material other than paper, the material other than paper may be a resin film, a nonwoven fabric, or the like. However, the packaging sheet 10 is particularly preferably made of paper without being laminated with a material sheet of a material other than paper, from the viewpoint of plastic reduction and/or from the viewpoint of being able to impart a natural texture unique to paper to a product. Although in general a paper packaging sheet is relatively easily torn, the sealing strength of a sealing portion can be appropriately adjusted by an application pattern of a heat sealing agent and the relationship between pressed portions and portions to which the heat sealing agent is applied according to the present embodiment, and an individually packaged absorbent article having sufficient sealing properties and prevented from being torn when unsealed can be provided.

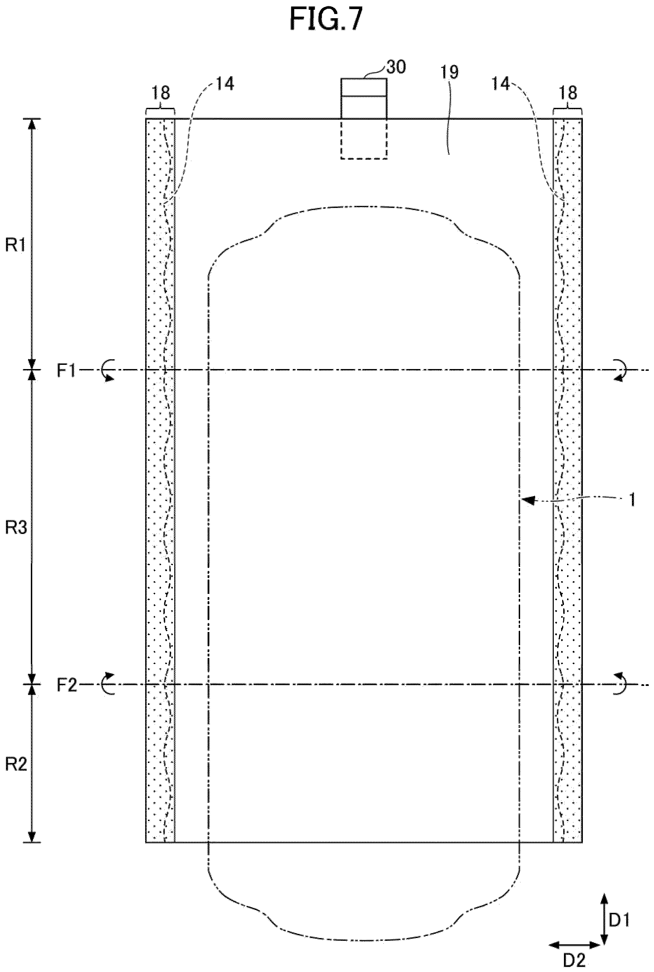

As long as an appropriate sealing strength can be secured in the sealing portion in the present embodiment, the paper packaging sheet 10 may be subjected to some processing other than the application of the heat sealing agent described above. The processing may include, for example, processing for physically deforming the packaging sheet 10, such as creping, embossing, calendering, slitting, or plying, or processing for adding a substance to the packaging sheet 10, such as water repellent processing, mold release processing, or ink printing processing. However, when the processing for adding the substance is performed, it is preferable to perform the processing on a region other than the region corresponding to the sealing portion in order to avoid an influence on the sealing strength of the sealing portion. Furthermore, it is preferable that the processing of adding the substance be performed on a region where the heat sealing agent is not provided, which is formed on the inner surface of the packaging sheet 10 (an example of such a region will be described later with reference to FIG. 7).

The antifouling function can be enhanced by the above-mentioned water repellent treatment or release treatment (for example, an application of a water repellent agent or a release agent containing a silicone-based resin, a paraffin-based resin, a fluororesin, or the like). In the case where the side of the packaging sheet 10 facing the displacement-prevention adhesive portion on the back surface of the absorbent article 1 (the inner surface of the packaging sheet) is subjected to water repellent processing, the displacement-prevention adhesive portion can be brought into contact with the packaging sheet 10 without a release sheet.

In addition, ink may be printed on the outer surface of the packaging sheet 10 in the present embodiment. The design of the individually packaged absorbent article is improved by performing ink printing. In the case where the ink printing is performed on the packaging sheet 10, it is preferable to use a material (uncreped paper or unembossed paper) that is not subjected to a process of forming irregularities on the surface of the packaging sheet, such as creping or embossing, as the packaging sheet 10.

A basis weight of the packaging sheet 10 used in the present embodiment may be preferably 8 g/m² to 50 g/m², more preferably 10 g/m² to 40 g/m² or less. By using the packaging sheet 10 having a basis weight in the above range, an individually packaged absorbent article having a good texture with a suppressed stiff feel can be obtained, and the generation of noise during carrying, unsealing, and the like can be prevented. The thickness of the packaging sheet 10 (the thickness after creping in the case of crepe paper) may be preferably 30 µm to 200 µm, and more preferably 35 µm to 100 µm.

The dimensions of the packaging sheet 10 may be determined according to the size and shape of the absorbent article 1 to be packaged. For example, the dimension of the longitudinal direction D1 (sometimes simply referred to as a "length") may be 100 mm to 450 mm, and the dimension of the lateral direction D2 (sometimes simply referred to as a "width") may be 70 mm to 250 mm, when the packaging sheet 10 is completely unfolded (not folded). In the example illustrated in the drawings, the packaging sheet 10 has a rectangular shape in an unfolded state, but may have a shape such as an oblong shape.

### (Packaging Structure)

As illustrated in FIGS. 1 through 4, the absorbent article 1 is packaged by being folded together with the packaging sheet 10, and an individually packaged absorbent article 100 is formed. When being folded, as illustrated in FIG. 2, the absorbent article 1 is placed on the inner surface of the packaging sheet 10 so that the back sheet side of the absorbent article 1 faces the inner surface of the packaging sheet 10. Then, the packaging sheet 10 and the absorbent article 1 are folded together in the longitudinal direction D1 along a first fold line F1 and a second fold line F2 along the width direction D2. More specifically, a first region R1 including one end 11 of the packaging sheet 10 in the longitudinal direction D1 is folded back in the longitudinal direction D1 along the first fold line F1, and a second region R2 including the other end 12 of the packaging sheet 10 in the longitudinal direction D1 is folded back along the second fold line F2. The region between the first region R1 and the second region R2 of the packaging sheet 10 is a third region R3. In the illustrated example, the packaging sheet 10 is folded in such a manner that the second region R2 is folded first and the first region R1 is folded to overlap the outer surface of the second region R2 (FIGS. 1 and 2), but the order of folding the first region R1 and the second region R2 may be reversed. In this way, after the absorbent article 1 is packaged by the packaging sheet 10 which is roll-folded into three sections together with the absorbent article 1, both edge portions of the lateral direction D2 are sealed along the longitudinal direction D1, and sealing portions 15, 15 are formed.

Such a packaging folded into three or more sections can be formed relatively simply, and it is possible to package the absorbent article 1 hygienically and to easily take out the absorbent article 1, with such a packaging. The way of folding is not limited to folding in three, and may be folding in four or more, or may be folding in two.

### (Sealing Portions)

As illustrated in FIG. 1, in the individually packaged absorbent article 100, the places where the absorbent article 1 is not present, which are located at both edges of the lateral direction D2, are sealed to form the sealing portions 15, 15. The sealing portions 15, 15 may be formed over the entire length of the longitudinal direction D1 of the packaging sheet 10. This is preferable from the viewpoint that dust, dirt, a finger, or a small object can be prevented from entering from the end edge in the lateral direction (namely, sealing properties can be obtained). The sealing portions 15, 15 may be formed by, for example, inserting both edge portions of the folded packaging sheet 10 (both edge portions of the lateral direction D2 of the packaging sheet 10) through a pair of rolls, and pressing and heating the edge portions with the rolls to press layers of the folded packaging sheet 10 against each other in the thickness-wise direction.

The range of the lateral direction D2 in which the sealing portion 15 is provided may be 20 mm from the end edge of the lateral direction D2. The sealing portion 15 may be formed in the entire range or may be formed in a part of the range, for example, at a position away from the end edge of the lateral direction D2. The length (width) of the sealing portion 15 in the lateral direction D2 is preferably 3 to 15 mm.

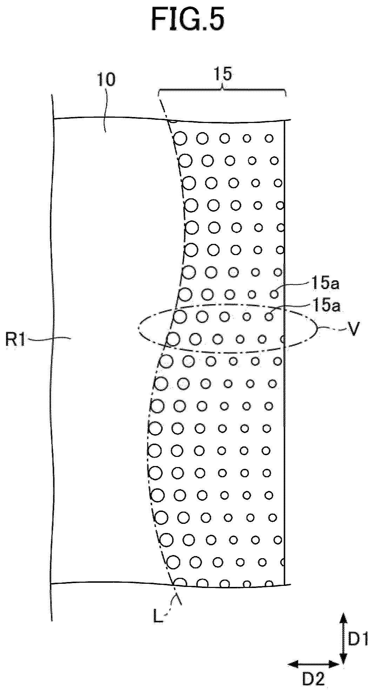

FIG. 5 is an enlarged view of portion III including the sealing portion 15. As illustrated in FIG. 5, the sealing portion 15 may include a plurality of pressed portions 15a, 15a, ... which are separated from each other in a plan view. The pressed portions 15a, 15a, ... are formed by being sandwiched and pressed between a patterned roll having a plurality of protrusions each having a shape corresponding to the pressed portions 15a, 15a, ... on the surface of the roll and a pattern-less roll (an anvil roll or the like) having no irregularities on the surface of the roll facing the patterned roll. Therefore, the pressed portions 15a, 15a, ... can be said to be portions where the fold of the packaging sheet is pressed in the thickness-wise direction, or pressed marks. The pressed portion 15a may be a portion recessed from its periphery (a portion protruding from its periphery when viewed from the upside-down). In the example illustrated in the drawings, the portions other than the plurality of pressed portions 15a, 15a, ... are non-pressed portions where the layers of the folded packaging sheets are not pressed against each other by the above-described protrusions on the roll surface. In this way, the pressed portions 15a, 15a, ... are scattered so as to be separated from each other in the surface direction, and the non-pressed portions are formed between the pressed portions 15a, 15a, ...; it is thus possible to avoid the layers of the folded packaging sheet from being continuously bonded to each other over the entire sealing portion 15, and to prevent the sealing strength from being excessively increased. Therefore, when the layers of the packaging sheet are peeled off from one another at the time of unsealing, the seal can be easily released while the tearing of the packaging sheet 10 is suppressed. In addition, since the air that has entered the inside is discharged to the outside through the non-pressed portions by applying pressure to the individually packaged absorbent article 100, it is possible to prevent the individually packaged absorbent article from being unintentionally ruptured before the package is unsealed. Furthermore, it is possible to prevent both edge portions of the individually packaged absorbent article 100 from becoming excessively hard and the texture from being impaired.

In the example illustrated in FIG. 5, a plurality of rows of the pressed portions 15a, 15a, ... arranged in a straight line along the lateral direction D2 are formed, and these rows are arranged in the longitudinal direction D1. The positions of the pressed portions 15a located on the innermost side of the lateral direction D2 are not aligned with each other in the lateral direction D2, and the inner contour line L of the sealing portion 15 is formed in a wavy line shape (see also FIG. 1). The wave-shaped inner contour line L of the sealing portion 15 improves the design of the sealing portion 15 and enhances the aesthetic property of the individually packaged absorbent article 100. The inner contour line L of the sealing portion 15 may be a line obtained by smoothly connecting in the longitudinal direction D1 the inner ends of the pressed portions 15a located on the innermost side of the lateral direction D2.

In the example illustrated in FIG. 5, the shape of each pressed portion 15a is circular, and the areas of the pressed portions 15a vary within one row of the plurality of pressed portions 15a arranged in the lateral direction D2. In the illustrated example, the areas of the pressed portions 15a increase toward the inner side of the lateral direction D2. This can increase the strength of the innermost portion of the lateral direction D2 corresponding to the peeling start position when the sealing portion 15 is released, and thus can prevent unintended unsealing before use.

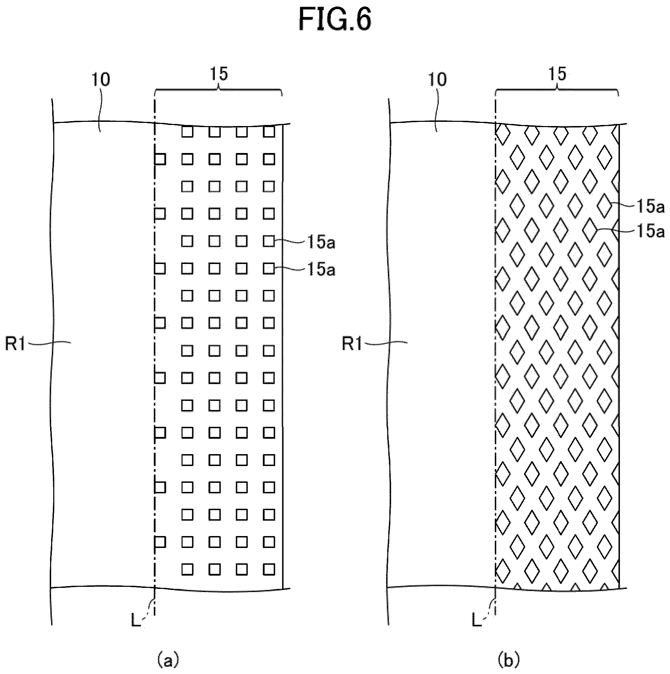

In the present embodiment, the sealing portion 15 is constituted by a plurality of pressed portions 15a, 15a, ... which are spaced apart from each other, and the arrangement, size, shape, and the like of the pressed portions 15a, 15a, ... are not particularly limited as long as the pressed portions 15a, 15a, ... overlap the heat sealing agent as described later. For example, as illustrated in FIG. 6(a), one pressed portion 15a may be a square, or as illustrated in FIG. 6(b), one pressed portion 15a may be a rhombus. The pressed portions 15a, 15a, ... may be arranged in a lattice pattern (FIG. 6(a)), in a staggered pattern (FIG. 6(b)), or in a random pattern. Further, even in the case of a lattice or zigzag arrangement, the number of the innermost pressed portions 15a, 15a, ... in the lateral direction D2 may be reduced (FIG. 6(a) and the like).

The shape of one pressed portion 15a is not limited to the shapes illustrated in FIGS. 5 and 6, and may be, for example, an ellipse, a square, a quadrangle other than a rhombus, a polygon other than a quadrangle, a heart shape, a star shape, a drop shape, or the like. In the sealing portion 15, the size of one pressed portion 15a may be a circle having a radius of about 0.2 mm to 1.5 mm or a size having an area equivalent thereto.

The area ratio of the pressed portions 15a, 15a, ... in the sealing portion 15, that is, the ratio of the total of the areas occupied by the pressed portions 15a, 15a, ... to the area the sealing portion 15, may be preferably 5% to 50%, and more preferably 10% to 40%.

### (Heat Sealing Agent)

In the present embodiment, a heat sealing agent is used to increase the sealing strength of the sealing portions 15, 15. The heat sealing agent may contain a thermoplastic resin, preferably a resin that softens or melts at 70 °C to 170 °C, and the heat sealing agent is preferably an olefin-based heat sealing agent. The heat sealing agent may be a water dispersion of resin particles. Specific examples of the heat sealing agent include "Chemipearl (registered trademark)" series manufactured by Mitsui Chemicals, Inc.

By using the heat sealing agent, even when the packaging sheet 10 is made of a difficult-to-fuse material having layers of the folded packaging sheet can be bonded to each other by sealing using heat (heat sealing). The heat sealing agent arranged between the layers of the packaging sheet is melted or softened to be brought into close contact with the packaging sheet, and then cooled to be solidified, whereby the layers of the folded packaging sheet are strongly fixed to each other. Therefore, a higher sealing strength can be obtained as compared with sealing utilizing physical deformation of the packaging sheet. Depending on the type of the heat sealing agent, the basis weight, the material of the packaging sheet, and the like, the sealing strength may be excessively high. However, in the present example, the heat sealing agent is applied in a predetermined pattern, and thus the sealing strength can be favorably adjusted.

For example, as illustrated in FIGS. 2 through 4, the heat sealing agent 20 may be applied to the inner surface of the packaging sheet 10. In this case, the range in which the heat sealing agent 20 is applied may be the entire inner surface or a part of the inner surface, but is a range including at least a region corresponding to the sealing portions 15, 15 (FIG. 1). More specifically, the regions corresponding to the sealing portions 15, 15 (sealing portion corresponding regions) may be regions 14 where the sealing portions 15 are to be formed (sealing-portion-to-be-formed regions), as illustrated in FIG. 7. FIG. 7 is a development view of the individually packaged absorbent article 100 similar to the one illustrated in FIG. 2, but the outline of the absorbent article 1 is illustrated with a two-dot chain line. As illustrated in FIG. 7, the heat sealing agent 20 is applied to at least the edge regions 18, 18 including the above-described sealing portion corresponding regions (sealing-portion-to-be-formed regions 14, 14). The edge regions 18, 18 may be regions having a constant width, and are regions that include the entire sealing-portion-to-be-formed region 14 and that do not overlap the absorbent article 1 in the state of the individually packaged absorbent article 100.

In the region 19 other than the edge regions 18, 18, the heat sealing agent 20 may be applied at a basis weight smaller than the basis weight of the heat sealing agent 20 in the edge regions 18, 18, or substantially not applied as in the example illustrated in FIG. 7. This makes it possible to reduce the cost of the heat sealing agent while the sealing strength of the sealing portion 15 is ensured. On the other hand, since some types of the heat sealing agents 20 have an effect of reinforcing the packaging sheet 10, it is possible to increase the strength of the packaging sheet 10 by increasing the area to which the heat sealing agent 20 is applied. In a case where a material that is easily torn, such as paper, is used as the packaging sheet 10, from the viewpoint of increasing the strength of the packaging sheet 10 itself, it is preferable to apply the heat sealing agent 20 also to the region 19 other than the edge regions 18, 18, and it is more preferable to apply the heat sealing agent 20 to both of the edge regions 18, 18 and the other region 19 at the same basis weight.

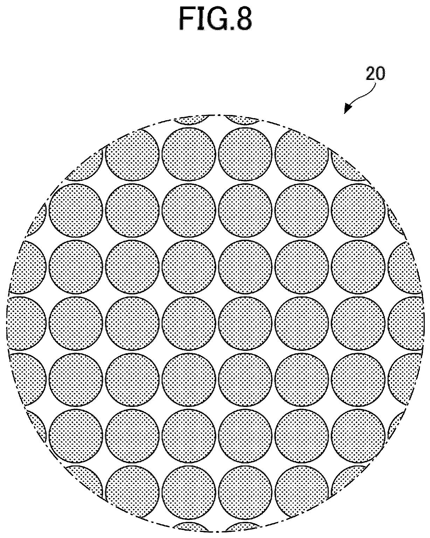

The predetermined pattern of the heat sealing agent 20 applied to the packaging sheet 10 is not particularly limited, but may be a pattern in which the heat sealing agent 20 is dispersed in a dotted manner, for example. FIG. 8 is an enlarged view of portion IV in FIG. 2. In the example illustrated in FIG. 8, the heat sealing agent 20 is arranged in a size-varying dots pattern. Since the heat sealing agent is applied in a size-varying dots pattern, the heat sealing agent can be dispersed and arranged in a regular pattern, and thus the heat sealing agent can be uniformly dispersed in the application region of the heat sealing agent, and the sealing strength of the sealing portion 15 can be prevented from becoming non-uniform. In addition, although the rigidity of the packaging sheet 10 changes and the tactile sensation or the like changes to a greater or lesser extent due to the applied heat sealing agent 20, it is also possible to suppress the change in the tactile sensation or the like (for example, to suppress the loss of flexibility of the packaging sheet 10) by making the pattern discontinuous.

In the example illustrated in FIG. 8, the heat sealing agent 20 is printed at a dot percentage of 70%. In the size-varying dots pattern, microscopically, dots of the heat sealing agent may be arranged separately from each other to be discontinuous, or dots of the sheet sealing agent may be arranged in contact with each other to be continuous.

The application area ratio of the heat sealing agent 20 in the application region of the heat sealing agent 20 is not particularly limited as long as the formation of the sealing portions 15, 15 by heat sealing is possible, but may be preferably 40% or more, more preferably 50% or more, further preferably 60% or more, and still further preferably 70% or more. When the application area ratio is 40% or more, the area of the heat sealing agent 20 extending on the packaging sheet 10 increases, and thus the sealing strength of the sealing portion 15 can be improved. The ratio can be 95% or less, 90% or less, or 80% or less, depending on conditions such as the type and basis weight of the heat sealing agent 20, the material of the packaging sheet 10, and the pattern and the area ratio of the pressed portion 15a (FIGS. 5 and 6, etc.) of the sealing portion 15. In the case of the size-varying dots pattern, the above-mentioned application area ratio of the heat sealing agent is the above-mentioned dot percentage. More specifically, the application area ratio of the heat sealing agent 20 can be a ratio of the total area of the heat sealing agent 20 in the edge region 18 to the area of the edge region 18 (described above) of the inner surface of the packaging sheet 10 before being folded. The case where the application area ratio is 100% indicates the case where the heat sealing agent 20 is applied in a solid manner.

Examples of the means for applying the heat sealing agent 20 include flexographic printing, offset printing, screen printing, and gravure printing. Among these, flexographic printing is preferable because a stable application amount and application pattern can be obtained, and an inexpensive aqueous ink can be used.

### (Relationship between Heat Sealing Agent Pattern and Pressed Portions)

As described above, the sealing portion 15 formed by sealing after folding the packaging sheet 10 includes the pressed portions 15a, 15a, ... and the pressed portions 15a can be formed at a predetermined area ratio in the sealing portion 15. The heat sealing agent 20 is formed in a predetermined pattern on the packaging sheet 10 before being folded, and the heat sealing agent 20 is applied at a predetermined application area ratio in the application region of the heat sealing agent 20. Herein, in order to reliably form the sealing portion 15 of the individually packaged absorbent article 100, it is important to arrange the heat sealing agent at the position of the pressed portions 15a when the edge portion of the lateral direction D2 of the folded packaging sheet is heated and nipped by the pair of rolls at the time of forming the sealing portion 15.

In the present embodiment, when the application region of the heat sealing agent 20 in the inner surface of one packaging sheet 10 is viewed, the pressed portion 15a overlaps the heat sealing agent 20. The sealing portion 15 is formed at the edge of the lateral direction D2 after the packaging sheet 10 is folded, and the portion corresponding to the sealing portion 15 (the portion included in the sealing portion 15) includes a portion where the inner surface of the packaging sheet 10 faces and a portion where the inner surface and the outer surface of the packaging sheet 10 face each other (a portion where the inner surface of the first region R1 and the outer surface of the second region face, FIGS. 1 and 4, etc.). Even at the location where the inner surface and the outer surface of the packaging sheet 10 face each other, as long as the region of the pressed portion 15a overlaps at least the heat sealing agent 20 applied to the inner surface of the packaging sheet 10 as in the present embodiment, overlapping of the region directly heated and heated by the roll and the heat sealing agent 20 is ensured, and reliable seal formation is therefore possible.

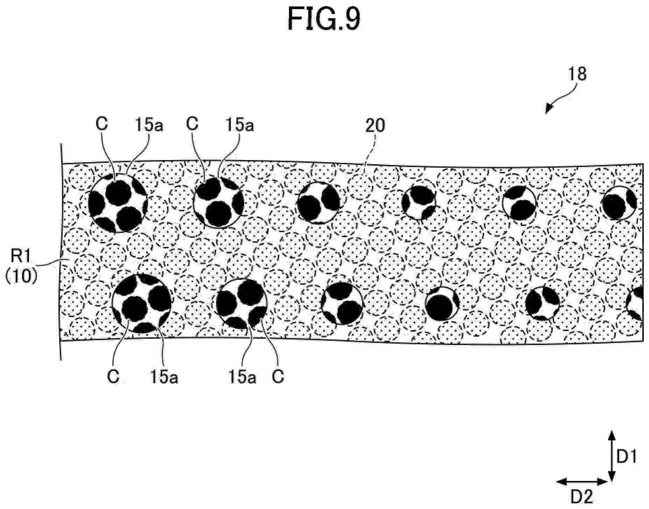

FIG. 9 is an enlarged view of one packaging sheet 10 (first region R1) included in portion V of FIG. 5. In FIG. 9, the heat sealing portion 20 applied to the inner surface of the first region R1 is indicated by a gray color surrounded by dotted lines, and the outlines of the pressed portions 15a, 15a are indicated by solid lines. The overlapping portion C where the pressed portion 15a overlaps the heat sealing agent 20 is illustrated in black. The overlapping portion C can be said to be a part common to the heat sealing agent 20 and the pressed portion 15a in one packaging sheet 10.

When viewed from the inner surface of one packaging sheet 10, for example, the ratio Pc (Pc = Sc/Sc × 100) of the sum (Sc) of the areas of the overlapping portions C to the sum (Se) of the areas of the pressed portions 15a in the inner surface of the first region R1 may be preferably 50% or more, 60% or more, or 70% or more, and may be 95% or less, 90% or less, or 80% or less. When the ratio Pc is 50% or more, the sealing strength can be secured, and sufficient sealing properties can be obtained before the individually packaged absorbent article is unsealed. In addition, when the ratio Pc is 95% or less, the packaging sheet can be smoothly unsealed without applying a load to the packaging sheet at the time of unsealing, and the ease of unsealing is enhanced. Since the packaging sheet can be prevented from being torn, a packaging sheet which is relatively easily torn, such as paper, particularly paper having a small basis weight, can be used. In the pressed portion pattern and the pattern of the heat sealing agent according to the example illustrated in FIG. 9, the ratio Pc of the sum (Sc) of the areas of the overlapping portions C to the sum (Se) of the areas of the pressed portions 15a is about 61.5%.

By setting the ratio Pc of the sum Sc of the areas of the overlapping portions C to the sum Se of the areas of the pressed portions 15a (also referred to as "the area ratio of the overlapping portions C") within a predetermined range, the sealing portions 15, 15 can be formed with an appropriate sealing strength regardless of the pattern (the shape and size of one pressed portion, interval, pitch, and arrangement of one pressed portion) and area ratio of the pressed portions 15a, 15a, and the pattern (the shape and size of the applied heat sealing agent in a plan view, whether the applied heat sealing agent is continuous or discontinuous) and applied area ratio of the heat sealing agent 20. In other words, if the ratio Pc is set to a predetermined range, the degree of freedom of the pattern of the heat sealing agent 20 and/or the pattern of the sealing portion 15 is also increased.

From the viewpoint of enhancing the sealing strength, as illustrated in FIG. 9, it is preferable that all the pressed portions 15a, 15a, ... at least partially overlap the heat sealing agent 20, but the pressed portion 15a which does not overlap the heat sealing agent 20 may be present. However, it is preferable that two or more pressed portions 15a (pressed portions 15a not including the overlapping portion C) not overlapping the heat sealing agent 20 be not adjacent to each other, that is, at least one of any two adjacent pressed portions 15a, 15a overlap the heat sealing agent 20. This can prevent a local decrease in the sealing strength. In other words, it is possible to prevent unevenness in the sealing strength of the sealing portion 15.

As illustrated in FIG. 9, when the heat sealing agent 20 is formed in in a size-varying dots pattern, the pitch of the size-varying dots of the heat sealing agent 20 (the length between the center of one dot and the center of a dot adjacent to the one dot) is preferably smaller than the pitch of the pressed portions 15a, 15a (the length between the center of one pressed portion 15a and the center of a pressed portion 15a adjacent to the one pressed portion 15a). This reduces the presence of the pressed portion 15a that does not have the overlapping portion C.

The ratio Pc of the sum (Sc) of the areas of the overlapping portions C to the sum (Se) of the areas of the pressed portions 15a can be measured by, for example, observing the inner surface of one packaging sheet 10 in an enlarged manner using a microscope or the like or capturing an image and analyzing the image after peeling off the sealing portion 15 of the individually packaged absorbent article 100.

### <Modified Examples>

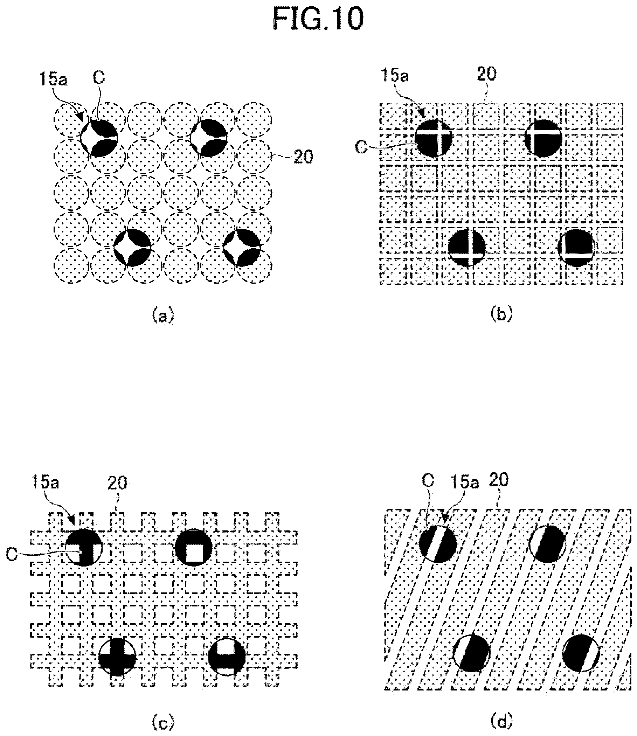

FIG. 10 illustrates modified examples of the overlapping portions C. In FIG. 10(a), the pattern of the heat sealing agent 20 is the same as that illustrated in FIG. 8. The pressed portions 15a, 15a, ... in FIG. 10(a) have the same size. In the example illustrated in FIG. 10(a), the ratio Pc of the sum (Sc) of the areas of the overlapping portions C to the sum (Se) of the areas of the pressed portions 15a is 62.61%.

As illustrated in FIG. 10(b), even in the case of a pattern in which the heat sealing agent 20 is arranged in a dot shape, the shape of the dots constituting the pattern may be a square. In this case, the application area ratio of the heat sealing agent 20 can be increased while the discontinuity of the heat sealing agent 20 is maintained. When the application area ratio of the heat sealing agent 20 is high, even if the same pattern of the pressed portions 15a, 15a, ... is used, it becomes easy to increase the ratio (the above-described Pc) of the overlapping portion C between the heat sealing agent 20 and the pressed portion 15a. On the other hand, since the heat sealing agent 20 is discontinuous, it is possible to suppress a change in a tactile sensation or the like of the packaging sheet 10 which may be caused by the application of the heat sealing agent 20 as described above. In the example illustrated in FIG. 10(b), the ratio Pc of the sum (Sc) of the areas of the overlapping portions C to the sum (Se) of the areas of the pressed portions 15a is 68.73%.

As illustrated in FIG. 10(c), the heat sealing agent 20 may be arranged in a lattice shape. The example of FIG. 10(c) can be said to be a pattern in which the heat sealing agent 20 is arranged in a manner that a plurality of lines extending along each of the longitudinal direction D1 and the lateral direction D2 intersect with each other. Such a continuous pattern of the heat sealing agent 20 can improve the strength of the packaging sheet 10 over the entire packaging sheet 10, and is therefore suitable for the case where a material that is easily torn, such as paper, or a material having a small basis weight is used as the packaging sheet 10. In the example illustrated in FIG. 810(c), the ratio Pc of the sum (Sc) of the areas of the overlapping portions C to the sum (Se) of the areas of the pressed portions 15a is 65.35%.

Furthermore, the application pattern of the heat sealing agent 20 may be a stripe shape in which stripes of the heat sealing agent 20 are arranged to be separated from each other. In this case, the extending direction of the stripe may be along the longitudinal direction D1 or the lateral direction D2, or may be oblique to the longitudinal direction D1 or the lateral direction D2 as illustrated in FIG. 10(d). In the example illustrated in FIG. 10(d), the ratio Pc of the sum (Sc) of the areas of the overlapping portions C to the sum (Se) of the areas of the pressed portions 15a is 67.26%.

### <Modified Examples of Application of Heat Sealing Agent>

As described above, the individually packaged absorbent article 100 according to the present embodiment may have a structure in which the packaging sheet 10 is folded into three or more sections. In this case, zones in which the number of overlapping layers of the packaging sheet 10 varies depending on the location may be formed. For example, in the three-fold packaging structure, as illustrated in FIGS. 1 and 4, a thick zone Za in which the packaging sheet 10 is layered in three and a thin zone Zb in which the packaging sheet 10 is layered in two are formed. When the sealing portion 15 is formed by, for example, heating and pressing with a pair of rolls, the sealing strength in the thick zone Za tends to be larger than the sealing strength in the thin zone Zb.

In contrast, the area ratio Pc of the overlapping portions C in the packaging sheet 10 included in the thick zone Za can be made relatively small, and the area ratio Pc of the overlapping portions C in the packaging sheet 10 included in the thin zone Zb can be made relatively large. This can suppress variations in the sealing strength over the longitudinal direction D1 of the sealing portion 15. Therefore, it is possible to prevent the packaging sheet 10 from being torn due to a load applied to the packaging sheet 10, which is caused by a change in the sealing strength in the middle of peeling the sealing portion 15 at the time of unsealing, and to suppress a sense of discomfort such as being caught at the time of unsealing. Therefore, in the present example for example, the application area ratio of the heat sealing agent 20 applied to the packaging sheet 10 included in the thick zone Za may be relatively small, and the application area ratio of the heat sealing agent 20 applied to the packaging sheet 10 included in the thin zone Zb may be relatively large.

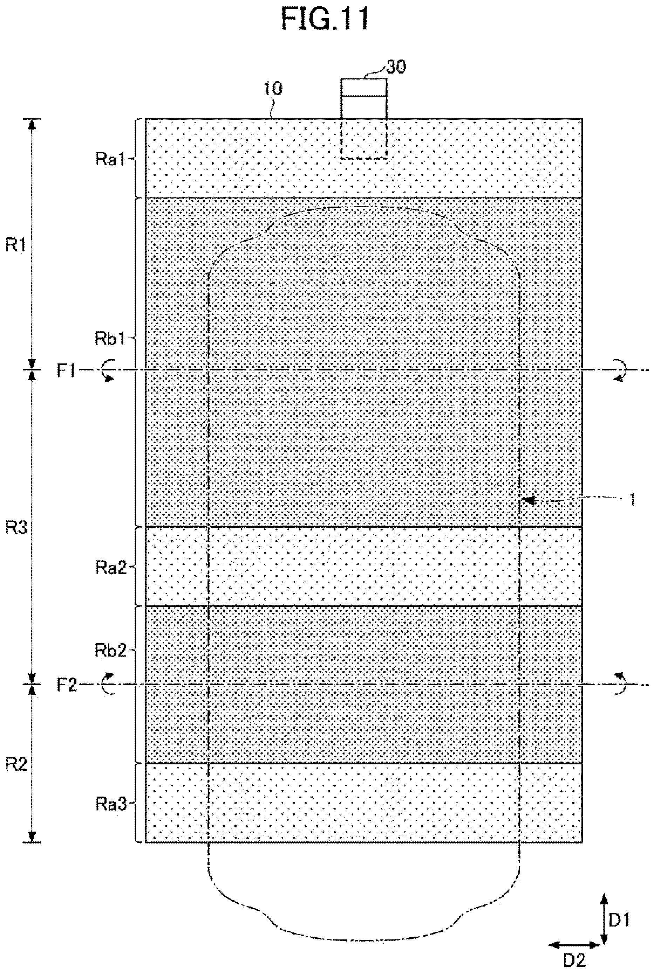

A more specific configuration is illustrated in FIG. 11. FIG. 11 is a development view of the individually packaged absorbent article 100 similar to the one illustrated in FIG. 2, but the outline of the absorbent article 1 is illustrated with a two-dot chain line. As illustrated in FIG. 11, the heat sealing agent 20 is applied to the entire inner surface of the packaging sheet 10, but the application area ratio of the heat sealing agent 20 varies depending on the location. The application area ratio of the heat sealing agent 20 in the regions Ra1 and Ra2 of the packaging sheet 10 corresponding to the thick zone Za can be made smaller than the application area ratio of the heat sealing agent 20 in the regions Rb1, Rb2, and Ra3 of the packaging sheet 10 corresponding to the thin zone Zb.

The present invention has been described above based on the embodiments, but the present invention is not limited to these embodiments. The above-described embodiments can be variously changed, modified, replaced, added, deleted, combined, and the like within the scope described in the claims, and these also belong to the technical scope of the present invention. The above-described components can be discretionarily combined.

Preferred aspects of the present invention will be further described below.

A first aspect is an individually packaged absorbent article in which an absorbent article is individually packaged by a packaging sheet made of a difficult-to-fuse material, wherein the packaging sheet is folded toward an inner surface side in a first direction of the packaging sheet, both edge portions in a second direction orthogonal to the first direction are sealed to form a sealing portion, the sealing portion includes a plurality of pressed portions separated from each other in a surface direction in which the packaging sheet is pressed in a thickness direction, a heat sealing agent is applied in a predetermined pattern to at least a region corresponding to the sealing portion of the inner surface of the packaging sheet, and the pressed portion overlaps the heat sealing agent applied to the packaging sheet.

In the first aspect, since the heat sealing agent is applied to at least the region corresponding to the sealing portion on the inner surface of the packaging sheet, even if the packaging sheet is made of a difficult-to-fuse material, a heat sealing can be used as a means for sealing both edge portions. Therefore, a higher sealing strength can be secured as compared to a seal that utilizes physical deformation (formation of irregularities or the like) of the packaging sheet without using an adhesive substance. The sealing strength may be excessively increased depending on the type, the basis weight, and the like of the heat sealing agent; however, according to the present aspect, since the heat sealing agent is formed in a predetermined pattern, the sealing strength can be appropriately adjusted. Furthermore, since the pressed portion overlaps the heat sealing agent applied to the inner surface of the packaging sheet, the sealing portion can be more reliably formed with more appropriate sealing strength regardless of the pattern of the pressed portion and the application pattern of the heat sealing agent.

In a second aspect, a ratio of a total area of overlapping portions where the pressed portions overlap the heat sealing agent to a total area of the pressed portions is 50 to 95%.

According to the second aspect, the ratio of the total area of the portions where the pressed portion overlaps the heat sealing agent (the part common to the pressed portion and the heat sealing agent) to the total area of the pressed portion is within a predetermined range, and thus the above-described effect that a sealing portion having an appropriate sealing strength can be formed regardless of the pattern of the heat sealing agent and/or the pattern of the pressed portion of the sealing portion can be improved. In other words, it is possible to improve the effect of obtaining a sealing portion which has sufficient sealing properties before the individually packaged absorbent article being unsealed and which can be easily peeled off without the packaging sheet being torn at the time of unsealing.

In a third aspect, the difficult-to-fuse material is paper.

According to the third aspect, paper has a unique natural texture (appearance and touch), and can provide comfort to users. The use of paper can also contribute to achievement of sustainable development goals. Although paper is generally a material that is easily torn, according to the present aspect, even in the case of a packaging sheet using such a material, a sealing portion having an appropriate sealing strength can be formed.

In a fourth aspect, the predetermined pattern of the heat sealing agent is a size-varying dots pattern.

According to the fourth aspect, the heat sealing agent is microscopically dispersed in the application region of the heat sealing agent, and thus the heat sealing agent can be more uniformly arranged in the application region. Therefore, for example, it is possible to prevent the sealing strength from being decreased due to an increase in the number of the pressed portions not overlapping the heat sealing agent.

In a fifth aspect, an application area ratio of the heat sealing agent in the packaging sheet is 40% or more.

According to the fifth aspect, the heat sealing agent is applied at a predetermined application area ratio, and thus the sealing strength in the sealing portion can be further increased. In addition, the packaging sheet itself is reinforced by the heat sealing agent, and even in the case where a packaging sheet that is easily torn (a packaging sheet made of paper, a packaging sheet having a small basis weight, or the like) is used, it is possible to suppress the packaging sheet from being torn at the time of unsealing.

In a sixth aspect, at least one of any two adjacent pressed portions overlaps the heat sealing agent.

According to the sixth aspect, it is possible to avoid an increase in the size of a portion that is not heatsealed regardless of the pattern of the heat sealing agent and/or the pattern of the pressed portion, and it is possible to prevent a local decrease in the sealing strength.

In a seventh aspect, a basis weight of the heat sealing agent in a region other than a region corresponding to the sealing portion is smaller than a basis weight of the heat sealing agent in a region corresponding to the sealing portion of the packaging sheet, or the heat sealing agent is not substantially applied in the region other than the region corresponding to the sealing portion.

According to the seventh aspect, an amount of the heat sealing agent in the region of the packaging sheet other than the region corresponding to the sealing portion in the individually packaged absorbent article is reduced, and thus the cost of the heat sealing agent can be reduced while the effect that the sealing portion has an appropriate sealing strength is maintained.

In an eighth aspect, the packaging sheet has a thin zone in which the packaging sheet is folded into two sections and a thick zone in which the packaging sheet is folded into three or more sections, and the application area ratio of the heat sealing agent in the packaging sheet included in the thick zone is smaller than the application area ratio of the heat sealing agent in the packaging sheet included in the thin zone.

In the individually packaged absorbent article, zones having different numbers of layers of a folded packaging sheet are formed, and the sealing strength tends to be higher in a zone having a larger number of layers (thick zone) than in a zone having a smaller number of layers (thin zone), and the sealing strength tends to be uneven over the longitudinal direction of the packaging sheet. In contrast, according to the eighth aspect, the basis weight of the heat sealing agent is changed according to the zone, and specifically, the application area ratio of the heat sealing agent of the packaging sheet included in the thick zone is made smaller and the application area ratio of the heat sealing agent of the packaging sheet included in the thin zone is made larger, whereby the sealing strength can be uniformized.

This application claims priority based on Japanese Patent Application No. 2023-055942 filed on March 30, 2023, the entire contents of which are incorporated herein by reference.

### REFERENCE SIGNS LIST

- 1: Absorbent article
- 3: Top sheet
- 4: Absorber
- 7: Side sheet
- 10: Packaging sheet
- 11: One end in the longitudinal direction
- 12: Other end in the longitudinal direction
- 15: Sealing portion
- 15a: Pressed portion
- 18: Edge region
- 19: Region other than the edge region
- 20: Heat sealing agent
- 30: Fastening tape
- 100: Individually packaged absorbent article
- C: Overlapping portion (a portion where a pressed portion overlaps with the heat sealing agent 20)
- D1: Longitudinal direction of the packaging sheet (first direction)
- D2: Lateral direction of the packaging sheet (second direction)
- F1: First fold line
- F2: Second fold line
- R1: First region
- R2: Second region
- R3: Third region
- Za: Thick zone
- Zb: Thin zone

## Claims

1. An individually packaged absorbent article in which an absorbent article is individually packaged by a packaging sheet made of a difficult-to-fuse material, wherein
a sealing portion is formed by folding the packaging sheet toward an inner surface side of the packaging sheet in a first direction, and sealing both edge portions of a second direction orthogonal to the first direction,
the sealing portion includes a plurality of pressed portions which are spaced apart from each other in a surface direction and which are formed by pressing the packaging sheet in a thickness direction,
a heat sealing agent is applied in a predetermined pattern to at least a region corresponding to the sealing portion on an inner surface of the packaging sheet, and
the pressed portion overlaps the heat sealing agent applied to the packaging sheet.

2. The individually packaged absorbent article according to claim 1, wherein
a ratio of a total area of overlapping portions where the pressed portion overlaps the heat sealing agent to a total area of the pressed portions is 50% to 95%.

3. The individually packaged absorbent article according to claim 1 or 2, wherein
the difficult-to-fuse material is paper.

4. The individually packaged absorbent article according to claim 1 or 2, wherein
the predetermined pattern of the heat sealing agent is a size-varying dots pattern.

5. The individually packaged absorbent article according to claim 1 or 2, wherein
an application area ratio of the heat sealing agent in the packaging sheet is 40% or more.

6. The individually packaged absorbent article according to claim 1 or 2, wherein
at least one of any two adjacent pressed portions overlaps the heat sealing agent.

7. The individually packaged absorbent article according to claim 1 or 2, wherein
a basis weight of the heat sealing agent in a region other than the region corresponding to the sealing portion is smaller than a basis weight of the heat sealing agent in a region of the packaging sheet corresponding to the sealing portion, or
the heat sealing agent is not substantially applied to a region other than the region corresponding to the sealing portion.

8. The individually packaged absorbent article according to claim 1 or 2, wherein
the individually packaged absorbent article includes a thin zone in which the packaging sheet is folded into two sections and a thick zone in which the packaging sheet is folded into three or more sections, and
an application area ratio of the heat sealing agent in the packaging sheet included in the thick zone is smaller than an application area ratio of the heat sealing agent in the packaging sheet included in the thin zone.
